# EUROPEAN PATENT APPLICATION

(11) **EP 1 872 758 A1**
(43) Date of publication of application: **02.01.2008**
(21) Application number: 06116089.1
(22) Date of filing: 26.06.2006
(51) Int. Cl.: A61F 13/15, A61F 15/00, B65D 85/16, B65D 75/38

(54) **Array of feminine hygiene products with colour coding**

(71) Applicant: The Procter and Gamble Company, Cincinnati, Ohio 45202 (US)
(72) Inventor: Adriaanse, Annemarie Elisabeth Josephine, Procter&Gamble AG CH-1213 Petit Lancy (CH); Rietzler, Miriam, 65812 Bad Soden (DE)
(74) Representative: L'Huillier, Florent Charles

(57) **Abstract**

An array of feminine hygiene articles (24a, 24b, 24c) wherein each feminine hygiene articles is associated with a specific colour coding (4a, 4b, 4c). The feminine hygiene articles comprise a primary package and individually wrapped absorbent articles (6a, 6b, 6c). The colour coding is present on the primary package and on the adhesive tape fastener (26) of the individual wrapper (28). The user can associate the colour coding with the functional characteristics (12, 14, 16, 18) of the absorbent articles without opening the individual wrapper. An elegant and aesthetic packaging system for feminine hygiene article is provided without additional costs.

## Description

### FIELD OF THE INVENTION

The invention relates to feminine hygiene articles such as sanitary napkins, also referred to as sanitary towels or pads. In particular, the invention relates to an improvement in the packaging of these articles.

### BACKGROUND OF THE INVENTION

Feminine hygiene articles are used by women to protect their undergarments from soiling by menstrual fluid, urine loss or vaginal discharges. These articles can be divided in those that are worn externally, commonly referred to as sanitary napkins (towels) or pads, and those inserted in the vagina, commonly referred to as tampons.

Sanitary napkins can be summarily described as substantially flat articles comprising a liquid-permeable layer (topsheet) which in-use faces the user, an absorbent element (sometimes called core) placed underneath the liquid-permeable surface, and a liquid-impermeable layer (backsheet) placed against the user's undergarment. Sanitary napkins are usually proposed as maxi (thick) or ultra (thin) pads.

Sanitary napkins are sold in a primary package (pack) normally containing several individually wrapped feminine hygiene articles. For example, it is common to have between 8 and 28 pads within a pack. Ultra pads are normally individually wrapped in a plastic wrapper. Because non-folded pads are usually 2-3 times longer than larger, it is usual to present them with both ends folded over the central portion. This folded configuration makes them easier to pack and carry, and also allows to use less material for the individual wrapper.

The individual wrapper is normally pressure and heat-sealed along the lateral sides of the folded pad, so as to protect the absorbent article from accidental contamination before it has been used, especially as the individual wrapper allows to carry one single unit separately from the primary packaging (pack). Some products have an adhesive tape fastener overlapping one of the edges of the individual wrapper, to allow re-sealing of the wrapper after it has been opened and the lateral side seals have been broken. One reason for this tape fastener is that it is common for women to re-use the individual wrapper to wrap and discard a used pad in a hygienic and discrete way. However the tape fastener does not provide a tight sealing comparable with the lateral heat sealing.

Manufacturers have multiplied the options and variants available to the users within each category of feminine hygiene articles. Pads are now generally available in various absorbency capacity (usually schematically shown by a number of drops displayed on the primary package), lengths, topsheet materials and with and without wings.

The articles contained within a primary package are normally identical. However it has become frequent for women to use a system combining different types of feminine hygiene articles during their menstrual period. For example, smaller or less absorbent articles may be preferred by a woman at the beginning and the end of her period, whilst more absorbent products will be used during the middle of the period when the flow is heavier or at night when it is not possible to change the pad for a period longer than usual. It is therefore not uncommon for a woman to have at her disposal different articles such as pads taken out of their respective primary packaging, for example in a bathroom drawer or in her handbag.

The individual wrappers are normally sealed to prevent contamination of the enclosed pads before use. Because these wrappers are normally made of an opaque plastic material, in the absence of indication on the individual wrapper itself, the user cannot recognize what type of article is present within the wrapper without opening it and destroying the seal. Even if it was possible to see through the wrapper, different variants will sometimes not be differentiable by visual inspection. For example it may not be possible for a user to tell the relative absorbent capacity of an article by superficially looking at it, as this property depends on the materials used for the construction of the pad or to tell if a product comes with or without wings.

Although it would be possible for a manufacturer to print a written notice on the individual wrapper to describe the product contained therein, this is not the choice usually adopted in the industry. In the currently marketed Always® Ultra pad range, each variant has a differently coloured individual wrapper: for example, in the UK, the current Always® Ultra Normal with wings pads are packaged in a deep green individual wrapper, whereas the Always® Ultra Normal pads (without wings) are packaged in light green packaging. The currently marketed products all feature an adhesive tape fastener, which is however identical across the array of products and does not provide a colour coding to differentiate between the variants.

The current pad packaging system suffers from the disadvantage that different individual wrappers must be manufactured, stored and used in the production of the pad for each variant, with an increase in cost and complexity compared to a situation where a single individual wrapper would be used. In addition, in products such as the Always® pads having a completely coloured individual wrapper, costs are further increased because ink is used to color the whole of the surface of individual wrapper.

A visual reference system for sanitary absorbent articles has been proposed in EP1,153,838A1. The reference system includes a scale of reference for certain characteristics such as absorbency and an element of information indicative of a certain level on the scale for the absorbent article. However such a system is relatively complicated and costly, and to the inventor's knowledge is not currently used in the industry. The reference system also takes a large space on the individual wrapper, which is detrimental to the aesthetical quality of the wrapper.

There is therefore a need for a cheaper packaging system for feminine hygiene articles, which allows the user to identify the content of an individual wrapper without having to open it. The system should also be aesthetic, discrete and simple to manufacture.

### SUMMARY OF THE INVENTION

The inventors have found that the adhesive tape fasteners commonly found on the individual wrappers of feminine hygiene products could be colour coded. The same colour coding is present on the primary package. By using the adhesive tape fastener and primary package to carry a colour coding, an elegant and aesthetic packaging system is provided without additional costs.

In particular, the present invention is directed to an array of feminine hygiene products as defined in claim 1, each of said feminine hygiene products comprising a specific colour coding on the primary package and on the adhesive tape fastener of the individual wrapper.

### BRIEF DESCRIPTION OF THE DRAWINGS

While the specification concludes with claims which particularly point out and distinctly claim the invention, it is believed the present invention will be better understood from the following description of preferred embodiments taken in conjunction with the accompanying drawings, in which like reference numerals identify identical elements and in which:
Fig. 1 is a perspective view of a primary package (pack) of pads, exemplary represented as Always® Ultra Normal product;
Fig. 2 is a view of the front panel of the packaging of Fig. 1;
Fig. 3 is an exemplary representation of an array of feminine hygiene products according to the invention, wherein the colour coding on the primary packaging differs for each product.
Fig. 4 is a front view of the absorbent articles (pads) in their wrapper corresponding to the products of Fig.3 represented immediately above, wherein the color coding of the primary packaging is also present on the adhesive tape fastener;
Fig. 5 shows one of the pads of Fig. 4 in an open configuration, the absorbent article being placed above the individual wrapper.

### DETAILED DESCRIPTION OF THE INVENTION

While the specification concludes with claims which particularly point out and distinctly claim the invention, it is believed that the invention will be better understood from the following description.

All percentages are by weight of total composition unless specifically stated otherwise. All ratios are weight ratios unless specifically stated otherwise. Unless explicitly stated otherwise, the term "comprising" is to be construed as open ended, meaning that other features, steps or ingredients can be added as long as they are suitable to be used in a feminine hygiene product.

The term "feminine hygiene products" as used herein refers to the articles of commerce sold for feminine protection, usually for menstrual and/or light incontinence control. These products are normally presented as a pack, referred hereinafter as primary package, containing several individually wrapped substantially identical absorbent articles, of the type commonly referred to as pads, pantiliners, liners, sanitary napkins or sanitary towels. By "substantially identical" we mean that the functional characteristics of the articles such as absorbency, length, shape, etc.. are the same within a given primary package. These products are normally disposable, which means that they are normally discarded after use, without being washed or reused.

The term "array of feminine hygiene products" designates a plurality of different feminine hygiene products marketed under the same brand name and belonging to the same category of feminine hygiene articles. It is common for manufacturers to market under the same brand name (e.g. Always®, Camelia®, Vania®, Kotex®) several variants in each category of feminine hygiene products. For example the currently marketed Always® Ultra pad array of product includes the NORMAL, NORMAL WITH WINGS, LONG, LONG WITH WINGS and NIGHT variants. The most common categories of feminine hygiene products for which an array of variant products are proposed include maxi (thick) pads, ultra (thin) pads, incontinence products and pantiliners. Maxi pads, also sometimes designated as maxi thick or thick pads, are relatively thick and bulky products and often do not comprise superabsorbent materials to absorb the fluid. Ultra pads, also sometimes designated as ultra thin or thin pads, are much thinner than maxi pads, usually less than 7 mm thin and comprise super absorbent polymers to provide the same absorbency as Maxi pads with a much smaller volume. Light and heavy incontinence products normally have a larger absorbency capacity than menstrual pads to accommodate a larger amount of fluid. Pantiliners are small and thin articles used to protect the undergarments against small amount of vaginal loss. These categories of products and their distinctions are well known.

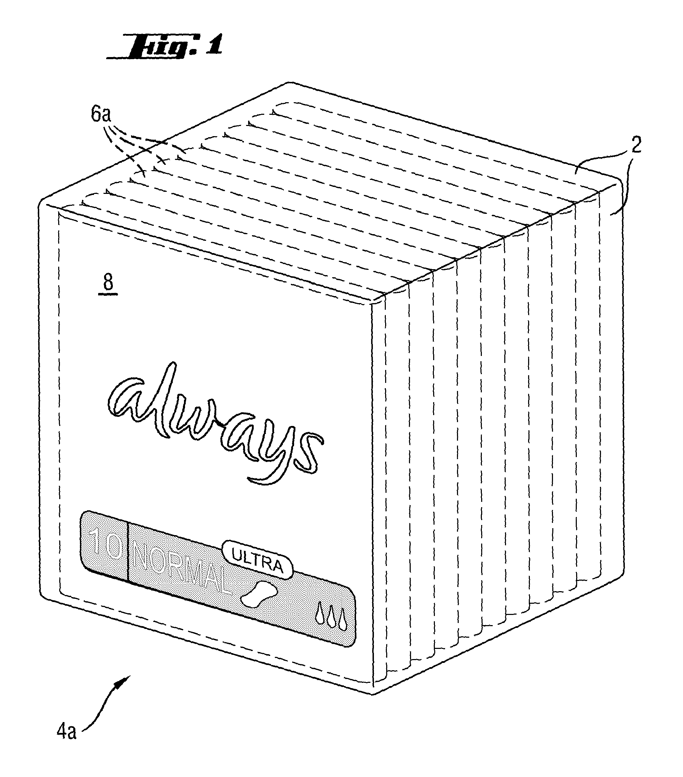

Referring now to Fig. 1, the primary package 2 of a feminine hygiene product 4a according to the invention is represented. This primary package has a generally cubic shape with a plurality of folded absorbent articles 6a (herein pads) stacked within, as is typical for this kind of packages. It is common to find between 8 to 28 absorbent pads per primary package, although a higher or lower number can also be proposed, for example at least 2. For example "value" pack are sometimes proposed which have twice the length of the standard primary package and accommodate twice the numbers of pads packaged in a standard pack. The primary package is normally made of a plastic material, typically polyethylene, but other materials may be used, e.g. cardboard or polypropylene.

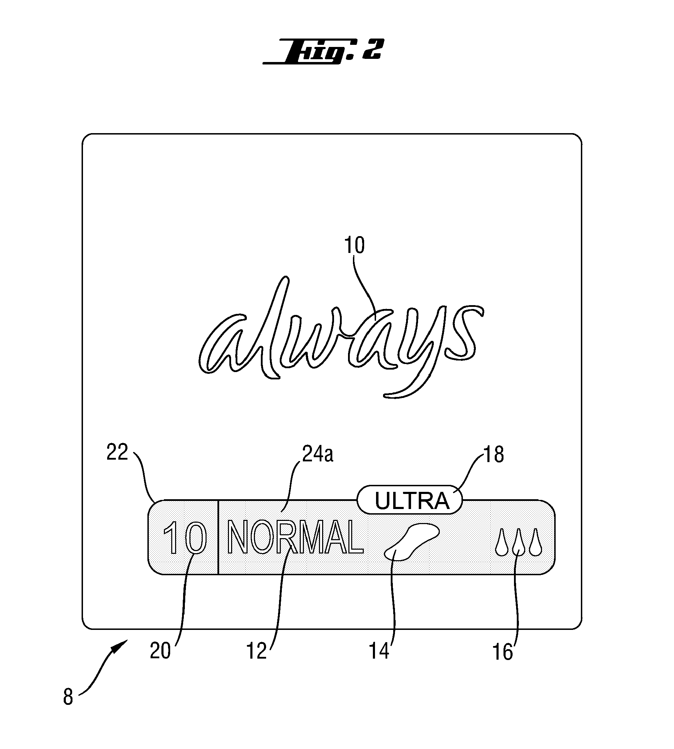

The primary package normally comprises on its outer surface various printed features, some of which are represented on Fig. 2 which represents the front panel 8 of the primary packaging 2. The brand of the product 10, as is exemplary represented by the tradename "Always", normally figures prominently on the front panel of the primary packaging. The functional characteristics of the absorbent articles packaged within the primary package are normally clearly indicated on the primary packaging to allow the consumer to buy the desired product.

Typical functional characteristics include indication of the length 12, the shape 14, the absorbency 16, the category of feminine hygiene product 18 and combinations thereof, of the absorbent products packaged within the primary package. There is currently no standard absorbency system adapted within the industry for pad type articles (unlike tampons), and each manufacturer will have its own reference system for the absorbency. The relative absorbency of each product is typically schematically represented by a number of droplets 16; rather than with an absorption capacity expressed in grams of liquid per article. An indication of the length of the article 12 may also be indicated on the primary package, either a direct measure, usually expressed in mm, or more often as a relative indication such as "NORMAL", "LONG". The shape of the absorbent article is also normally indicated, for example by a drawing or icon 14 representing the pad itself. Sometimes a description of the material used to make the absorbent article is indicated, in particular the material of the topsheet (e.g. cotton), or an indication of the feel of the topsheet (e.g. cottonfeel), if it considered better than the typical plastic material used for the topsheet. If the pad comprises side wings, this will normally be indicated, for example with a written indication such as "WITH WINGS" or "PLUS". The number of articles 20 within the primary package will normally also be indicated. Other functional characteristics include the presence of absence of a lotion or acent.

Usually, at least some or all the indications of the functional features 12, 14 ,16, 18 of the articles packaged within the primary package are grouped together on the front panel 8 of the primary package so as to be immediately understood by the user considering the product. These indications of the functional characteristics of the absorbent article are often grouped within an area delimited by a frame 22 to make them quickly and easily identifiable.

Design features or artwork reinforcing the attractiveness of the product (not represented) will normally be present on all the panels of the packaging. In commercial articles all the panels of the articles will usually comprise printed matter, such as artwork illustration, indication of the functional characteristics of the articles and/or a description of other articles within the array which are also proposed for sale.

In the present invention, each feminine hygiene product within the array of feminine hygiene products is associated with a specific colour coding. The colour coding is different for each different feminine hygiene product. This colour coding is present on the primary package and on the adhesive tape fastener.

Another product variant within the array of products will be associated with a different colour coding. The array of feminine hygiene products comprises at least two different feminine hygiene products (variants), preferably at least three different feminine hygiene products.

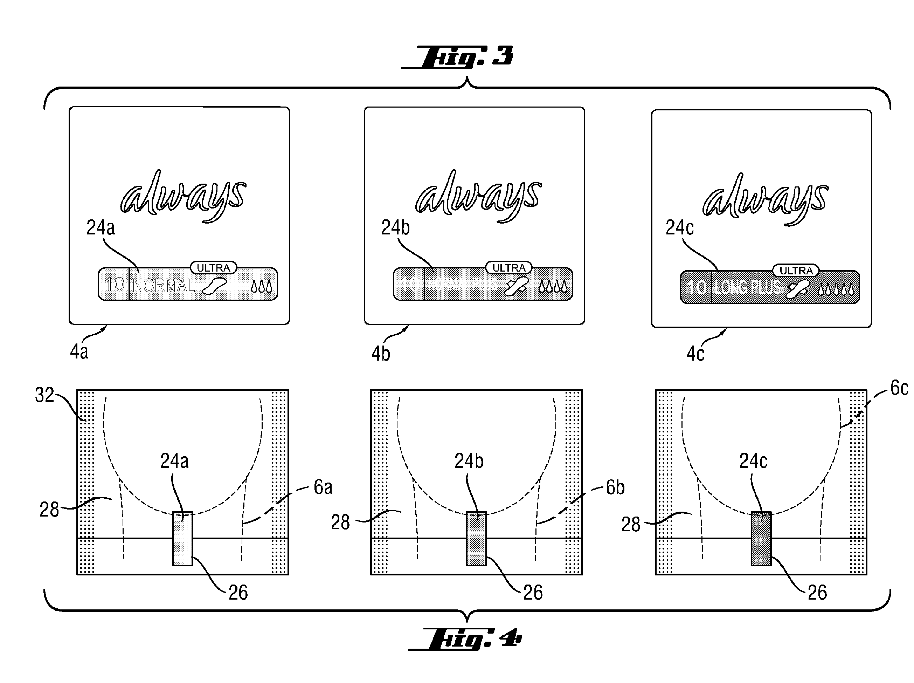

Figure 3 shows an array of three feminine hygiene products 4a, 4b, 4c, herein exemplary represented as Always® Ultra products. The front panel of each primary package comprises a specific colour coding 24a, 24b, 24c, which is present within the frame 22 grouping the functional characteristics of the absorbent article. The colour coding is materialized in this embodiment by a coloured background within the frame. Any colours may be used for the colour coding, such as any shades of green, blue, orange, red, yellow or even shades of black. An example could be dark green for the Normal product, light green for the Normal Plus product and dark purple for the Long Plus.

The colour coding 24a, 24b, 24c may take the form of a printed area visible on the surface of the primary package. Said area is printed in a specific colour, which is associated with the absorbent article 6a, 6b, 6c packaged therein. The colour coding on the primary package may be indicated in other places than the frame grouping the indication of the functional characteristics of the absorbent articles, for example the whole or part of the outer surface of the primary package may be printed with an ink representing the desired colour coding. A pigmented plastic material may also be used to make the primary packaging and provide the colour coding. It is also possible to print the brand name 10 of the array of feminine hygiene article products with an ink having the specific colour coding, or print the functional indications in the specific colour coding for the specific variant considered.

It was found advantageous to use the area within the frame 22 which groups the indication of the product characteristics 12, 14, 16, 18 to display the colour coding 24a, 24b, 24c. The area within the frame may be printed in a colour associated with the specific absorbent article packaged therein, whereas another variant article will be printed in a different colour.

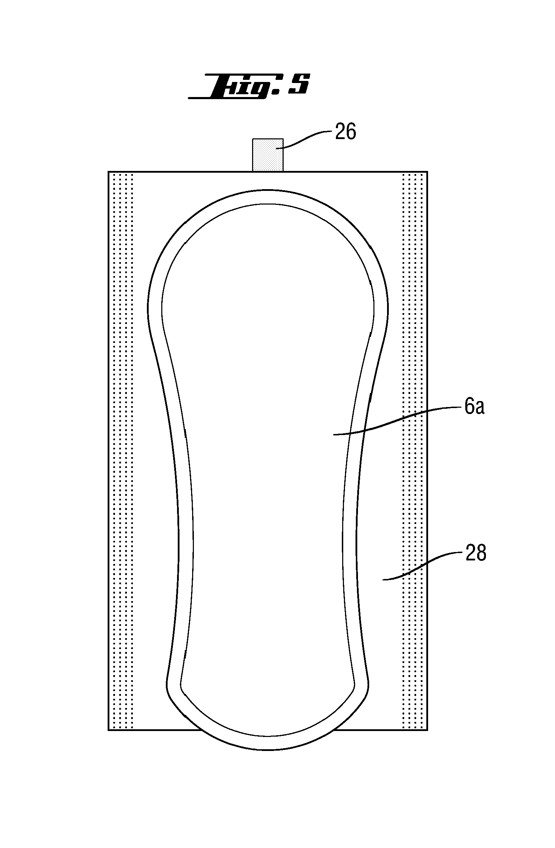

Referring now to Fig. 4, there are represented three folded pad 6a, 6b, 6c in their individual wrapper, each pad corresponding to the respective feminine hygiene product 4a, 4b, 4c represented immediately above. Each folded pad 6a, 6b, 6c is packaged in a secondary packaging 28, which will be referred to hereinafter as an individual wrapper. The individual wrappers represented are made of the same material for economy, although different materials made be sued. One of these pads is represented in its open configuration on Fig. 5, with the lateral heat-seals 32 of the individual wrapper broken, wherein the unfolded pad 30 is now visible and disposed across the individual wrapper 28.

The individual wrapper 28 is normally made of a plastic material, such as polyethylene, but of course other material may be used such as polypropylene or paper. The individual wrapper may be heat-sealed along its lateral sides, usually in a series of rows of dots to allow easier breaking of the seal 32 than what would be obtained with a linear continuous heat-seal. There are two common ways to fold the absorbent article and individual wrapper. One is to fold the absorbent article and wrapper together: the absorbent article is disposed flat on the wrapper, and then both elements are folded together twice, a first end portion being first folded over the central portion of the pad, the second end portion being then folded over the first portion. Another is the so called "fold and wrap" method, wherein the absorbent article is folded twice similarly as in the previous method but independently of the wrapper, the folded absorbent article being then positioned on the wrapper, which wrapper is folded over the folded article and then heat-sealed. These two methods are well known. In both cases, the appearance of the pad in the folded configuration is similar to what is represented in Fig. 4.

The adhesive tape fastener 26 is normally a relatively small, thin, rectangular, strip of plastic material, coated on one side with an adhesive material. As represented on Fig. 4, the adhesive tape fastener may be adhesively fixed at about half its length over the edge of the uppermost folded section of the individual wrapper, the other half of the adhesive tape fastener being releasably adhered to the surface of the individual wrapper of the first folded section. The adhesive tape fastener comprises an adhesive composition, which may be coated on about only three quarter of the length of the adhesive tape fastener, so that half of the tape fastener is strongly bonded to one edge of the individual wrapper, while the other half presents a non bonding area which is makes it easier to grasp and detach the tape fastener from the surface of the individual wrapper to open and close the pad with this adhesive tape fastener. The dimension of the adhesive tape fastener of the invention may range from about 0,5 cm² to about 10 cm², usually from about 1 cm² to about 5 cm², more usually from about 1,5 cm² to about 4 cm². The shape may also be different from a rectangle.

The adhesive tape fastener is normally used as a means for sealing the individual wrapper before use and re-sealing it after the lateral seals 32 have been broken, for example to wrap a used product within the wrapper before discarding it. Adhesive tape fasteners are in currently marketed product normally white, although coloured adhesive tape fasteners have also been proposed. However, in the previously proposed products, the colour of the adhesive tape fastener was identical across the array of products. It has never been proposed to use differently coloured adhesive tape fastener to distinguish between different products within an array of feminine hygiene products.

As represented in Fig. 3 and Fig. 4, the colour coding 24a, 24b, 24c present on a the primary packaging of a given product 4a, 4b, 4c is also present on the adhesive tape fastener 26 of the individual wrapper 28 enclosing the absorbent product 6a, 6b, 6c in said primary packaging. Because the functional characteristics of the absorbent articles and the colour coding are indicated on the primary packaging, an association is made by the user between the functional characteristics of the absorbent articles and the specific colour coding when she buys the product. Thanks to this association of the functional characteristics of the articles with the colour coding on the primary package, the colour coding on the adhesive tape fastener immediately provides an indication to the user of the functional characteristics of the absorbent article packaged therein.

The colour coding on the adhesive tape fastener may simply be realized by using a coloured (e.g. pigmented or printed) plastic material for the adhesive tape fastener. The products of the invention may be manufactured by the usual manufacturing processes, and therefore do not require substantial investment costs. Another advantage of the claimed system is that the same sheet of individual wrapper material may be used for all the feminine hygiene products within the array of feminine hygiene products, resulting in substantially identical individual wrappers across the array. In addition to be economically more efficient, this also provides the array of articles with a homogenous design and look. Of course all individual wrappers may not look exactly the same, as these are normally cut from a larger piece of material printed with a pattern which is larger than the usual surface of each individual wrapper. This provides each individual wrapper with slight variations in their appearance, whilst keeping the individual wrappers' look relatively homogenous.

The primary packages may also be substantially identical across the array of feminine hygiene products, with only the colour coding, and of course, the functional indications varying across the array of products, again providing for a more aesthetically pleasing design and look across the array of products.

## Claims

1. An array of feminine hygiene products (4a, 4b, 4c), each of said feminine hygiene products comprising:
a) a plurality of substantially identical absorbent articles (6a, 6b, 6c), each of said absorbent articles being individually packaged in an individual wrapper (28), wherein an adhesive tape fastener (26) is present on the individual wrapper to allow opening and re-sealing of the individual wrapper,
b) a primary package (2), said primary package enclosing said plurality of individually packaged absorbent articles,
wherein the primary package comprises an indication of the functional characteristics (12, 14, 16, 18) of the absorbent articles packaged therein, such as absorbency, length, shape and combinations thereof,
**characterized in that** each of the primary packages within said array of feminine hygiene products comprises a colour coding (24a, 24b, 24c), said colour coding being also present on the adhesive tape fastener (26) of the individual wrapper (28) of said absorbent articles (6a, 6b, 6c),
said colour coding (24a, 24b, 24c) being different for each different feminine hygiene products (4a, 4b, 4c) within the array of feminine hygiene products.

2. An array of feminine hygiene products according to claim 1, wherein all individual wrappers across the array of feminine hygiene products are substantially identical.

3. An array of feminine hygiene products according to claim 1 or 2, wherein said indication of the functional characteristics (12, 14, 16 ,18) of the absorbent article is indicated on the front panel (8) of the primary package.

4. An array of feminine hygiene products according to claim 3 wherein at least several functional characteristics (12, 14 ,16 ,18) are indicated within a frame on said front panel and wherein said colour coding is present within said frame (22) on said front panel.

5. An array of feminine hygiene products according to claim 3 or 4, wherein at least some of said functional characteristics are indicated on the primary package over said a background of said colour coding.

6. An array of feminine hygiene products according to any of the preceding claims wherein the adhesive tape fastener (26) is made of a coloured material matching the colour coding of the primary packaging, preferably a coloured plastic material.

7. An array of feminine hygiene products according to any of the preceding claims, wherein the feminine hygiene products belong to a category selected from maxi pads, ultra pads, incontinence products and pantiliners, preferably selected from maxi pads and ultra pads, more preferably ultra pads.

8. An array of feminine hygiene products according to any of the preceding claims wherein the array comprises at least three different feminine hygiene products (4a, 4b, 4c).

9. An array of feminine hygiene products according to any of the preceding claims wherein each primary package individually comprises at least 2 individually packaged absorbent articles, preferably from 8 to 28.

10. An array of feminine hygiene products according to any of the preceding claims wherein the adhesive tape fasteners have a dimension ranging from about 0,5 cm² to about 10 cm².
